# EUROPEAN PATENT APPLICATION

(11) **EP 4 364 652 A1**
(43) Date of publication of application: **08.05.2024**
(21) Application number: 22205508.9
(22) Date of filing: 04.11.2022
(51) Int. Cl.: A61B 5/021, A61B 5/0235

(54) **AIR VENT FOR A HEMODYNAMIC MEASUREMENT APPARATUS**

(71) Applicant: Koninklijke Philips N.V., 5656 AG Eindhoven (NL)
(72) Inventor: KUNT, Stanislav, Eindhoven (NL); FALK, Jonas, Eindhoven (NL)
(74) Representative: Philips Intellectual Property & Standards

(57) **Abstract**

The present invention relates to an air vent (150) for a hemodynamic measurement apparatus (200), the hemodynamic measurement apparatus (200) comprising an inflatable cuff (110) and an inflator (120), the air vent (150) comprising an air vent body (160) configured to be disposed in a flow path of fluid (130) between the inflatable cuff (110) and the inflator (120) or at a bladder (112) of the inflatable cuff (110), the air vent body (160) having one or multiple orifices (153) arranged in the air vent body (160), wherein the one or multiple orifices (153) are configured to let fluid pass from the flow path of fluid (130) or from the bladder (112); and a cover (155) covering the one or multiple orifices (153) and being configured to permanently let fluid pass from the one or multiple orifices (153) to the atmosphere, wherein the cover (155) is configured and arranged to redirect the flow of fluid from the one or multiple orifices (153) and to protect the one or multiple orifices (153) from material entering the one or multiple orifices (153) from the atmosphere.

## Description

### FIELD OF THE INVENTION

The present invention relates to an air vent for a hemodynamic measurement apparatus, to a hemodynamic measurement apparatus and a hemodynamic measurement system.

### BACKGROUND OF THE INVENTION

Hemodynamic measurements are used in many medical examinations to assess the condition of a subject. The availability of hemodynamic monitoring ensures optimal tissue perfusion and oxygen delivery while maintaining adequate mean arterial blood pressure of the patient. For example, a commonly conducted hemodynamic measurement is the measurement of blood pressure (BP) but may include measurement of other parameters of the functional characteristics of the heart and the circulatory system that affect the perfusion of tissues with oxygenated blood. BP can either be obtained invasively via an arterial blood-pressure catheter (ABP) or non-invasively via a BP inflatable cuff (NIBP).

Continuous advanced hemodynamic monitoring is available only to few patients. ABP allows for direct pressure measurement, blood sampling for hemodynamics monitoring and also medication titration. However, invasive BP monitoring is usually used only in the case of high-risk patients or in complex surgical procedures because it is associated with adverse effects, such as e.g. infections. The large majority of patients are monitored using traditional non-invasive technologies (e.g. NIBP, SpO2, ECG, respiration rate). On the other hand, the conventional NIBP technology supports only BP measurements and does not offer any additional hemodynamic parameter (e.g. cardiac output, stroke volume, etc.).

US 2015/359446 A1 discloses a blood pressure measuring system configured to surround a patient's body part, comprising pressurization means for applying pressure to the body part, and comprising a kinking-proof shell, wherein the kinking-proof shell is arranged so as to be located between the pressurization means and the body part, when the blood pressure measuring system surrounds the body part. Such a blood pressure measuring system is also referred to as Advanced Monitoring Cuff (AMC) which may be used for hemodynamic measurements.

Advanced Monitoring Cuffs may require a lower inflation rate compared to conventional inflatable cuffs (NIBPs). However, in e.g. a hospital a pneumatic system (e.g. an inflator or pump) that is used to inflate the inflatable cuff may only allow one set inflation rate and may therefore not be compatible with certain types of inflatable cuffs such as e.g. an Advanced Monitoring Cuff. Hence, the use of such inflatable cuffs may be restricted. A solution to adapt the inflation rate of the inflatable cuff is needed.

US 2021/0393151 A1 discloses a cuff for use with an inflation-based non-invasive blood pressure measurement apparatus. The cuff comprises a valve comprising a flow resistance such that the valve passes part of the flow of fluid received in the bladder to the atmosphere in order to inflate the bladder at a required flow rate for inflating the cuff.

### SUMMARY OF THE INVENTION

It is an object of the present invention to provide a less expensive and less complex solution that allows setting the inflation rate and, optionally, the deflation rate of the inflatable cuff to a desired value.

The invention is defined by the independent claims.

In a first aspect of the present invention, an air vent for a hemodynamic measurement apparatus comprising an inflatable cuff and an inflator is presented that comprises:
an air vent body configured to be disposed in a flow path of fluid between the inflatable cuff and the inflator or at a bladder of the inflatable cuff, the air vent body having one or multiple orifices arranged in the air vent body, wherein the one or multiple orifices are configured to let fluid pass from the flow path or from the bladder; and

a cover covering the one or multiple orifices and being configured to permanently let fluid pass from the one or multiple orifices to the atmosphere, wherein the cover is configured and arranged to redirect the flow of fluid from the one or multiple orifices and to protect the one or multiple orifices from material entering the one or multiple orifices from the atmosphere.

In a second aspect of the present invention, a hemodynamic measurement apparatus is presented that comprises
an inflatable cuff;
a hose configured to connect the inflatable cuff with an inflator; and
an air vent as disclosed herein.

The inflator may be arranged in a pneumatic system configured to inflate the inflatable cuff and the air vent may be arranged at any suitable location within the flow path of fluid or in connection with the flow path of fluid.

In a third aspect of the present invention, a hemodynamic measurement system is presented that comprises:
a hemodynamic measurement apparatus as disclosed herein; and
an inflator configured to inflate the inflatable cuff.

The inflator and the inflatable cuff may each be connected to e.g. a patient monitor or a blood pressure measurement device, such that the inflatable cuff is inflated by the inflator with a flow path of fluid via the patient monitor or blood pressure measurement device. It is also possible that the inflator is arranged within the patient monitor.

Preferred embodiments of the invention are defined in the dependent claims.

It has been found that certain inflatable cuffs, such as Advanced Monitoring Cuffs, used for hemodynamic measurements may require a specific inflation rate, and in particular a reduced inflation rate compared to conventional inflation. Other cuffs like conventional NIBP inflatable cuffs may require a specific (reduced) inflation rate to perform certain measurements as well. However, in e.g. a hospital a pneumatic system that is available to inflate the inflatable cuff may only allow one specific inflation rate and may therefore be compatible only with a specific type of inflatable cuff or may be useful for BP measurement only, but not for one or more other hemodynamic measurements. Hence, the use of various types of inflatable cuffs may be restricted and/or certain measurements and applications may not be possible.

The present invention is hence based on the idea to provide a new separate element, herein called "air vent", for use with a hemodynamic measurement apparatus, and to dispose the air vent in the flow path of fluid connecting the inflatable cuff to the inflator. The air vent is configured to permanently allow a certain amount of fluid (e.g. air or other gas) pass from the fluid path through one or more orifices within its air vent body to the atmosphere. The air vent thus allows to reduce the fluid flow to and/or from the inflatable cuff, wherein the size, arrangement and number of orifices of the air vent body and/or the cover may define the fluid flow rate from the flow path of fluid to the atmosphere. The (passive) air vent thus allows a volumetric flow rate (i.e., an amount of fluid bleeding through the air vent) that is dependent on the difference in pressure between the inside of the system and the atmosphere, i.e. the volumetric flow rate will not necessarily be held at a constant value.

Some pneumatic systems provide only a set or controllable pressure or inflation/deflation rate which can be used to inflate and/or deflate the inflatable cuff. Nevertheless, the lower limit of the inflation rate that can be provided by known pneumatic systems used for hemodynamic measurements is generally not low enough. The air vent according to the present invention allows to adapt the set inflation rate of the pneumatic system to a specific lower inflation rate for the inflatable cuff, i.e., the air vent may shift down the whole range of possible inflation rates. Furthermore, the deflation rate of the inflatable cuff may be increased by allowing a certain amount of fluid to escape through the air vent to the atmosphere, additionally to fluid flowing back to a e.g. a pneumatic device. Thus, the inflatable cuff may be deflated in a shorter time. However, the air vent does not necessarily have to allow an increase of the deflation rate but may in other embodiments allow only to decrease the inflation rate. Hence, the air vent according to the present invention allows at least to lower the inflation rate to a specific value, which makes it possible to use inflatable cuffs and/or make measurements requiring a certain inflation rate other than a set inflation rate given by a pneumatic system.

The cover covering the one or multiple orifices is arranged to redirect the flow of fluid from the one or multiple orifices to the atmosphere may allow to constrict the ingress of dirt, e.g. particles, liquid, or contaminations from the atmosphere into the orifices. Particles and/or liquid that have entered the one or more orifices might enter the inflatable cuff and/or the inflator via the flow path of fluid. Hence, particles or liquid should be hindered to enter the flow path of fluid via the one or multiple orifices. Also, particles or liquid could block the one or multiple orifices and thus the flow of fluid to the atmosphere. Therefore, the cover is configured and arranged to protect the one or multiple orifices, the flow path of fluid, the inflator and inflatable cuff from contamination.

The air vent according to the present invention thus provides a solution to flexibly use inflatable cuffs with a pneumatic system that provides a fixed inflation rate or pressure for inflation of the cuff. The air vent is inexpensive and simple to produce and may be suitable for use in a disposable product, where the cuff (including the air vent and the hose) will be exchanged for each subject and/or after a certain number of uses.

Preferably, the cover and the one or multiple orifices are configured and arranged to reduce noise caused by the flow of fluid though the air vent to the atmosphere. For example, by spreading the fluid flow over a larger area and/or changing the direction of the fluid flow one or several times, e.g. by a change of the cross section for the fluid flow, produced noise may be reduced. This may increase the comfort of the subject and the medical staff during a hemodynamic measurement. Hence, the cover and the one or multiple orifices may function as a muffler.

In an embodiment, the cover at least partially encloses the air vent body and comprises a plurality of pores permeable for fluid. Therefore, the cover may comprise porous material with a plurality of permeable pores. The pores may finely distribute the fluid flow from the one or multiple orifices to the atmosphere. Furthermore, the pores may block dirt from the atmosphere entering the one or multiple orifices and may therefore protect the air vent from contamination. Additionally, the pores may help to lower the risk of unintentional blockage of the air vent, which could prevent the fluid from flowing from the air vent into the atmosphere. The one or multiple orifices may e.g. comprise a diameter in the range of 0.01 to 0.25 mm, more particularly in the range of 0.05 to 0.2 mm, for example with an average size of 0.15 mm. The one or multiple orifices may also comprise diameters larger or smaller than the range given above. In an embodiment the air vent may comprise only one orifice having e.g. a diameter in the range of approximately 0.07 mm to 0.2 mm.

It shall be noted that the diameter is not the only parameter influencing the flow properties of an orifice. Its length should be considered as well. Volumetric flow rates over the pressure drop over the orifice may be defined, which may be a relevant property for the intended use of the air vent. Volumetric flow rates in the range of approximately 0.5 to 5 ml/s, more particularly in the range of 0.7 to 3 ml/s, and 20 to 750 mmHg, more particularly in the range of 30 to 500 mmHg may be used. In case of multiple orifices, the sum of flow rates should be in that range.

In another embodiment, the cover is arranged at a distance from the air vent body to form one or multiple openings between the air vent body and the cover letting the fluid pass to the atmosphere. Therefore, the one or multiple orifices may lead the fluid flow into a cavity between the cover and the air vent body, whereupon the fluid may flow to the atmosphere.

Preferably, the air vent body is configured as a hollow cylinder or pipe and the cover is ring-shaped and arranged around the air vent body. For example, the air vent body may have a similar shape of the cross section as the hose configured to connect the inflatable cuff with the inflator.

In an embodiment the multiple orifices are distributed over the circumference of the air vent body. Therefore, the fluid may flow from different directions from the air vent into the atmosphere. By distributing the fluid flow over a large area, the produced noise may be reduced, in particular compared to a valve.

In another embodiment, at least some of the axes of the multiple orifices intersect in one point of the air vent body, in particular in a center point of the air vent body, and/or are inclined at different angles with respect to a longitudinal axis of the air vent body. Therefore, the multiple orifices may be e.g. arranged radially, starting from the center point of the air vent body. The multiple orifices may be also arranged such that some of the axes do intersect in one point and some of the axes are inclined at different angles with respect to the longitudinal axis of the air vent body. For example, the longitudinal axis of the air vent body is parallel to the fluid flow direction through the air vent body. Such a configuration of the axes of the multiple orifices distributes the bleeding air to different directions, minimizing the impact of possibly covered parts of the air vent outlet (covered e.g. by blankets, sheets, clothes, etc.). The arrangement and configuration of the axes should not influence the basic flow parameters of the air vent.

Preferably, the air vent further comprises one or more spacers between the air vent body and the ring-shaped cover to form one or multiple channels between the outer surface of the air vent body and the cover. Therefore, one or more cavities between the air vent body and the ring-shaped cover may be formed.

In an embodiment, the ring-shaped cover is arranged in contact with the outer surface of the air vent body and is made of porous material. Therefore, the ring-shaped cover encloses the air vent body, and the fluid may flow from the one or multiple orifices through the porous material to the atmosphere. Furthermore, the porous material may block dirt from the atmosphere entering the one or multiple orifices due to the relatively small size of the pore openings compared to one larger opening. Therefore, the porous material may protect the air vent from contamination. Additionally, the porous material may help to lower the risk of unintentional blockage of the air vent, which could prevent the fluid from flowing from the air vent into the atmosphere. Covering of all the outer surface of the air vent body and thus all the pores may be less likely than a partially blockage of the air vent.

In another embodiment, the air vent body is configured as a plate or ring segment, and the cover is configured as a porous block arranged on top of the air vent body. Therefore, the cover may partially enclose the air vent body. For example, the cover only encloses the air vent body where the one or multiple orifices merge from the air vent body.

Preferably, the air vent further comprises a first connector arranged at a first end of the air vent and a second connector arranged at a second end of the air vent, the first connector and second connector being configured to insert the air vent into the flow path of the fluid. Therefore, the first and second connectors are configured to connect the air vent to the flow path of the fluid. The air vent may be detachably attached to e.g. a hose. For example, the first and the second connectors may be configured as barb connectors.

In an embodiment, the air vent is configured to be attached to one of an inlet hose of the inflatable cuff, a hose configured to connect the inflatable cuff to the inflator, a hose configured to connect the inflatable cuff to the inflator via a patient monitor, an outlet hose of the inflator, or a fluid connection within the inflator. Therefore, it is understood that the air vent may be attached to any suitable location in the flow path of fluid or in connection with the flow path of fluid. The hose may be configured as well to connect the inflatable cuff to a hemodynamic measurement apparatus, such as a BP measurement device.

In another embodiment, the inflatable cuff is a shell cuff, and the air vent is attached to the hose or at the bladder. Here, shell cuff refers to a cuff comprising a rigid, kinking-proof shell shaped spirally around the extremity of the subject, e.g. upper arm of a human, wherein the shell is configured to be pressed around the extremity during inflation and arranged between the bladder and the extremity of the subject. For example, the shell cuff is a cuff described as "Advanced Monitoring Cuff" in US 2015/0359446 A1.

### BRIEF DESCRIPTION OF THE DRAWINGS

These and other aspects of the invention will be apparent from and elucidated with reference to the embodiment(s) described hereinafter. In the following drawings
Fig. 1A shows an embodiment of a known cuff in a deflated state.
Fig. 1B shows an embodiment of the known cuff shown in Fig. 1A in an inflated state.
Fig. 2 shows a schematic diagram of an embodiment of a hemodynamic measurement system according to the present invention.
Figs. 3A, 3B and 3C show schematic diagrams of different arrangements of the air vent in the hemodynamic measurement system shown in Fig. 2.
Fig. 4 shows a schematic diagram of a first embodiment of an air vent according to the present invention.
Fig. 5 shows a schematic diagram of a second embodiment of an air vent according to the present invention.
Figs. 6A and 6B show a schematic diagram of another embodiment of an air vent according to the present invention comprising connectors.
Fig. 7A shows an assembly configured to measure a flow rate through the air vent to the atmosphere.
Fig. 7B shows a diagram of a flow rate and a pressure signal.

### DETAILED DESCRIPTION OF EMBODIMENTS

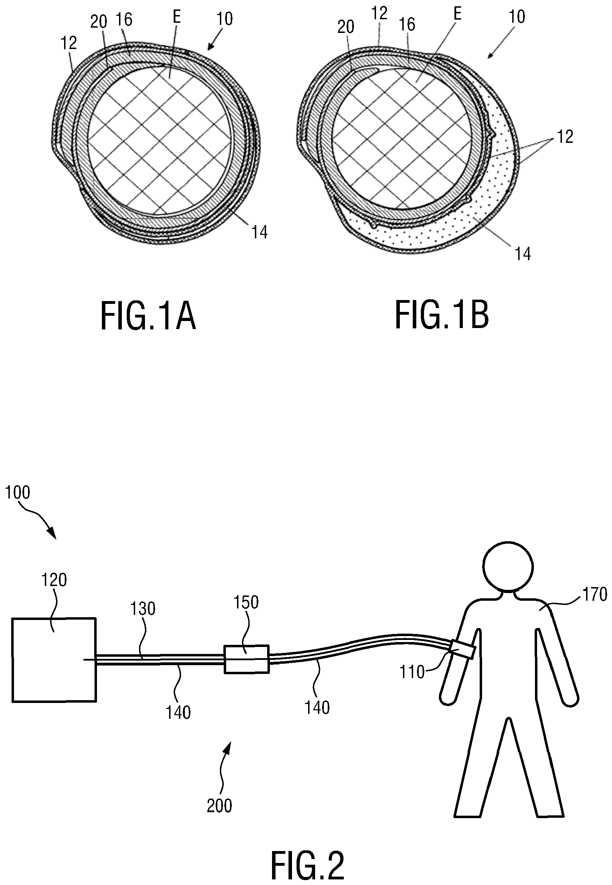

Fig. 1 shows an embodiment of a known inflatable cuff 10, as e.g. disclosed in US 2015/359446 A1, in a deflated state (Fig. 1A) and in an inflated state (Fig. 1B). The cuff 10 (also called Advanced Monitoring Cuff (AMC) herein) uses a kinking-proof shell 20 that is arranged between the pressurization means, comprising a pressure actuator 12 with an air/fluid bag 14, and the body part E (e.g. a subject's upper arm). The kinking-proof shell 20 is arranged (sandwiched) between the pressure actuator 12 with the air/fluid bag 14 and a flexible element 16.

The kinking-proof shell 20 is preferably made from plastic material, such as polyethylene. The thickness of the kinking-proof shell 20 is chosen so that the kinking-proof shell 20 does not buckle when pressure is applied to the air/fluid bag 14, while at the same time the kinking-proof shell 20 is flexible enough to allow for a certain deformation of the kinking-proof shell 20. That is, when pressure is applied to the air/fluid bag 14, overlapping edge regions of the kinking-proof shell 20 move or slide relatively to each other so as to reduce the diameter of the kinking-proof shell 20. However, the kinking-proof shell 20 thereby remains substantially ring-shaped. The kinking-proof shell 20 may comprise individually formed shell elements that are substantially in parallel to each other to better fit or adapt to the shape of the body part E.

Certain inflatable cuffs such as e.g. the Advanced Monitoring Cuff shown in Fig. 1, but also standard inflatable cuffs used for certain applications, may require an inflation rate other than a standard inflation rate provided by a pneumatic system. In particular, a lower inflation rate may be needed. However, the available pneumatic system may not provide an adjustable inflation rate and/or several different inflation rates. Hence, a solution to flexibly use inflatable cuffs with one sole pneumatic system is needed. Preferably, the solution should be easy to use and be suitable for the application as a disposable element. For example, in a hospital, the inflatable cuff and/or a hose configured to be attached to the inflatable cuff may be used as a disposable and may thus be frequently exchanged.

Fig. 2 shows a schematic diagram of a first embodiment of a hemodynamic measurement system 100 according to the present invention. It comprises an inflatable cuff 110 (e.g. an AMC), an inflator 120 configured to inflate the inflatable cuff 110 with a fluid, the fluid flowing along a flow path of fluid 130 from the inflator 120 to the inflatable cuff 110, and a hose 140. Hose 140 encloses the flow path of fluid 130, connecting inflator 120 with the inflatable cuff 110. The hemodynamic measurement system 100 further comprises an air vent 150 disposed in the flow path 130 of the fluid. Air vent 150 allows reducing the inflation rate of the inflatable cuff 110 by permanently letting pass fluid from the flow path of fluid 130 to the atmosphere (the surrounding environment) during inflation of the cuff 110. Compared to a mere deflation of the inflatable cuff 110 via the inflator 120, the air vent 150 may additionally (but not necessarily) increase the deflation rate by permanently letting pass a certain amount of fluid from the flow path of fluid 130 to the atmosphere during deflation of the cuff 110. In Fig. 2, the inflatable cuff 110 is wrapped around an upper arm of a subject 170, e.g. of a human. The inflatable cuff 110 may be also wrapped around other extremities of subject 170. Cuff 110 may be e.g. the Advanced Monitoring Cuff as shown in Figs. 1A and 1B. The inflatable cuff 110, the hose 140 and the air vent 150 together represent a hemodynamic measurement apparatus 200, which is part of the hemodynamic measurement system 100.

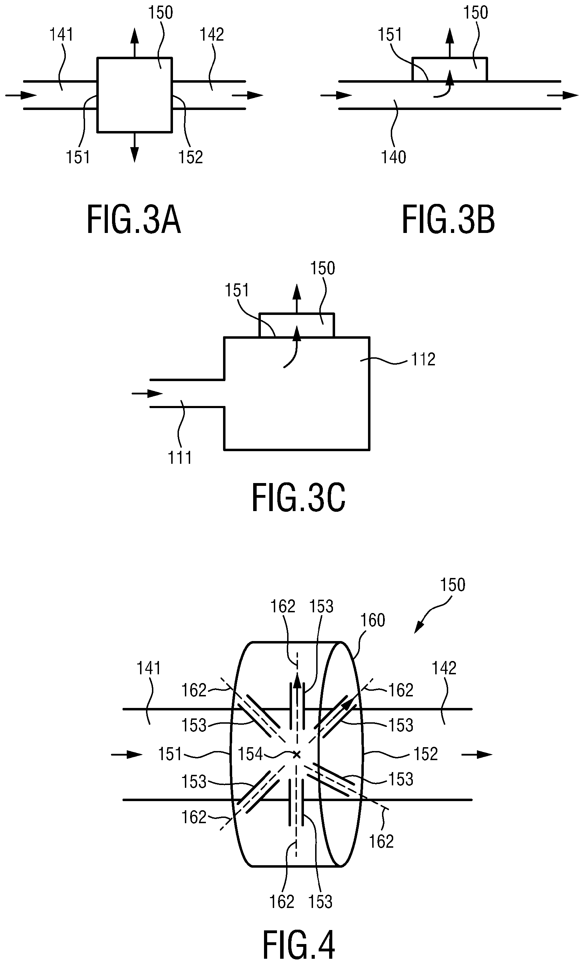

Figs. 3A-3C show schematic diagrams of different arrangements of the air vent 150 in the hemodynamic measurement system 100, according to which the air vent 150 is disposed at different exemplary locations within the hemodynamic measurement system 100.

In the embodiment shown in Fig. 3A, the air vent 150 is disposed in the flow path of fluid 130 between the inflatable cuff 110 and the inflator 120, letting fluid to pass from the inflator 120 to the inflatable cuff 110. The arrows indicate the flow direction of the fluid through the air vent 150. A first end 151 of the air vent 150 is attached a hose 141 connected to the inflator 120 and a second end 152 of the inflator is attached to a hose 142 connected to the inflatable cuff 110. For example, hose 142 is an inlet hose 111 of the inflatable cuff 110. During inflation, part of fluid flowing along the flow path of fluid 130 flows through the air vent 150 to the atmosphere, reducing the inflation rate of the inflatable cuff 110. During deflation, the direction of the two arrows in the hose 141, 142 is reversed and the fluid flows back from the inflatable cuff 110 to the inflator 120, wherein part of the fluid flowing back may flow to the atmosphere via the air vent 150. It shall be noted, however, that the air vent 150 does not necessarily have to increase the deflation rate of the inflatable cuff 150, but the air vent 150 may prevent fluid from flowing from the inflatable cuff 150 to the atmosphere. Hence, the air vent may be working only in one fluid flow direction.

In the embodiment shown in Fig. 3B, the air vent 150 is disposed in the flow path of fluid 130 between the inflatable cuff 110 and the inflator 120, being attached to a hose 140 connecting the inflator 120 with the inflatable cuff 110. Fluid flows from the flow path of fluid 130 into air vent 150 and out to the atmosphere. Due to the arrangement of the air vent 150 at the hose 140 rather than in the hose 140, the air vent 150 shown in Fig. 3B may not comprise a second end 152. Hence, the air vent 150 does not necessarily have to let a certain amount of fluid pass through the fluid flow path 130. Similarly to the embodiment shown in Fig. 3A, the air vent 150 reduces the inflation rate and may also increase the deflation rate of the inflatable cuff 110.

In the embodiment shown in Fig. 3C, the air vent 150 is attached to a bladder 112 of the inflatable cuff 110 and may thus let fluid within the bladder escape to the atmosphere during inflation and deflation.

The air vent 150 may be also disposed at any other suitable location within the hemodynamic measurement system 100 or the hemodynamic measurement apparatus 200, e.g. attached to an outlet hose 121 of the inflator 120, a fluid connection within the inflator 120 or a hose 140 configured to connect the inflatable cuff 110 to a patient monitor 170. For example, patient monitor 170 may comprise the inflator 120.

Fig. 4 shows an exemplary implementation of the air vent 150 according to the present invention. The air vent 150 comprises an air vent body 160 disposed in the flow path 130 of fluid. The arrows indicate the flow direction of fluid. The air vent body 160 comprises a first end 151 and a second end 152, each connected to the flow path of fluid 130, configured to let fluid pass from the inflator 120 to the inflatable cuff 110 and/or from the inflatable cuff 110 to the inflator 120.

In this implementation, the air vent body 160 is configured as a hollow cylinder or pipe and comprises multiple orifices 153 (e.g. bores or channels) configured to permanently let fluid pass from the flow path of fluid 130 or the bladder 112 of the inflatable cuff 110. It should be understood that air vent body 160 may also comprise only one orifice 153. In the embodiment shown in Fig. 4, the multiple orifices 153 are arranged such that the axes 162 of the multiple orifices 153 intersect in the center point 154 of the cylindrical shaped air vent body 160. The axes 162 of the multiple orifices 153 may be also arbitrarily arranged and/or only some of the axes 162 of the multiple orifices 153 may intersect in the center point 154.

The air vent 150 further comprises a cover (not shown) that covers the multiple orifices 153 and is configured to permanently let fluid pass from the multiple orifices 151 to the atmosphere. The cover is further configured and arranged to redirect the flow of fluid from the multiple orifices and to protect the orifices from ingress of e.g. particles or fluids, which could contaminate the air flow path 130 and thus the inflatable cuff 110 and/or the inflator 120. For example, the cover may comprise a porous material, e.g. in the form of a ring or cylinder of porous material arranged at the outer surface of the air vent 150. "Porous material" hereby means material comprising a plurality of pores. The cover and the multiple orifices 153 are configured and arranged to reduce noise pollution, e.g. by splitting the fluid flow, spreading the fluid flow over a large area around the air vent body 160 and/or changing the direction of fluid flow.

The cover may, at the same time, work as an ingress protection and a muffler by reduction of the acoustic noise (the hissing sound). The silencing effect is mainly caused by spreading the air flow over the area of the muffler and thus reducing the flow velocity as it is the major contributor to the acoustic noise level.

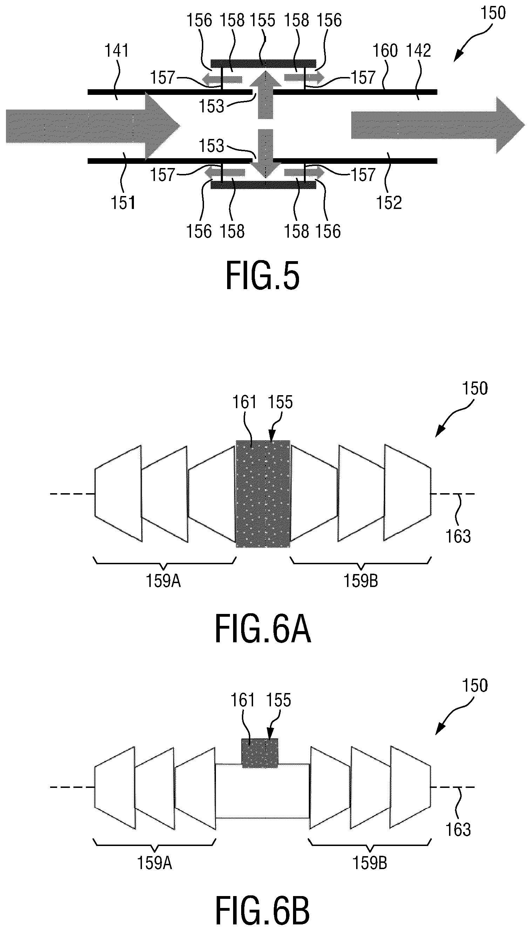

Fig. 5 shows another exemplary implementation of the air vent 150 according to the present invention. The arrows in Fig. 5 indicate the flow direction of fluid. The air vent body 160 is configured as hollow cylinder or pipe and comprises multiple orifices 153. The cover 155 is ring-shaped and arranged around the air vent body 160 at a distance from the air vent body 160 to form one or multiple openings 156 between the air vent body 160 and the cover 155. Between the air vent body 160 and the cover 155 one or more cavities are formed. The air vent 150 may further comprise one or more spacers 157 between the air vent body 160 and the ring-shaped cover 155 to form one or multiple channels 158 between the outer surface of the air vent body 160 and the cover 155.

Fig. 6A and 6B show embodiments of the air vent 150 according to the present invention comprising a first connector 159A at the first end 151 and a second connector 159B at the second end 152 of the air vent 150. The first connector 159A and the second connector 159B are configured as barb connectors. Each connector 159A, 159B may be plugged into a hose, e.g. the second connector 159B may be plugged into the inlet hose 111 of the inflatable cuff 110. The barb connector ensures that the air vent 150 is not accidentally detached from the hose 140. The air vent 150 may also comprise other type of connectors suitable to connect the air vent 150 to the flow path of fluid 130.

The connectors 159A, 159B allow to attach the air vent 150 detachably or non-detachably to e.g. hose 140, the inlet hose 111 of the inflatable cuff 110 or the outlet hose 121 of the inflator 120. Detachably attached air vents 150 provide the advantage that a user (e.g. hospital staff) may use air vents 150 with different flow rates to the atmosphere (e.g. air vents 150 with differing number and/or sizes of orifices 153 and/or with different number and/or sizes of channels 158 of the cover 155), allowing to inflate inflatable cuffs 110 at different inflation rates.

In the embodiment shown in Fig. 6A, the cover 155 is arranged as a ring around the air vent body and comprises a porous material (material comprising a plurality of pores 161, the pores 161 being indicated as dots in Fig. 6A and 6B), working as a muffler and ingress protection. In the embodiment shown in Fig. 6B, the cover 155 comprises a porous material as well, but is not completely arranged around the air vent body. Cover 155 is arranged in contact with the outer surface of the air vent body, e.g. shaped like a block with an offset from the longitudinal axis 163 of the air vent body or shaped as hollow semi-cylinder, etc.

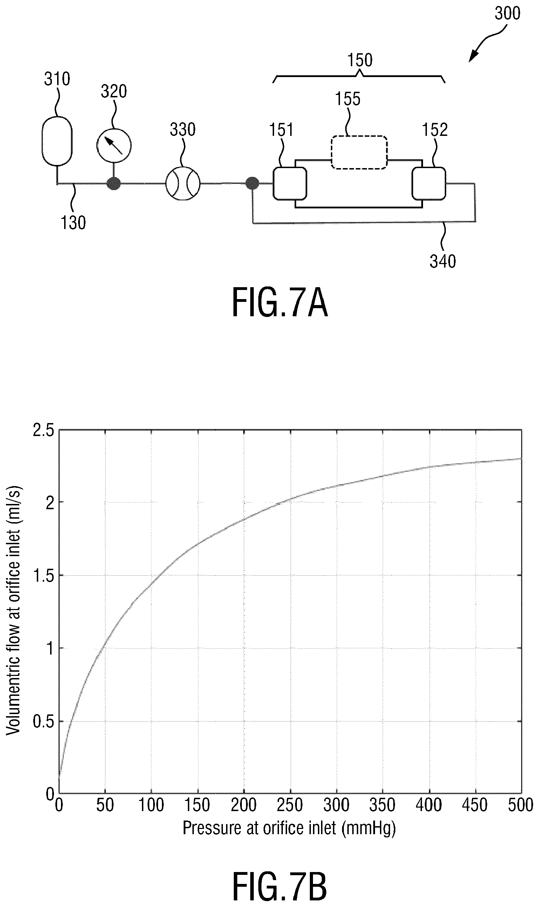

Fig. 7A shows an assembly 300 configured to measure the flow rate of fluid through the air vent 150 to the atmosphere. The assembly 300 is thus suitable for assessing how much fluid can flow through the air vent 150 to the atmosphere and therefore for assessing the capability of the air vent 150 to lower an inflation rate of the inflatable cuff 110. Similarly, the assembly 300 is suitable to assess the capability of the air vent 150 to increase the deflation rate of the inflatable cuff 110. The flow rate of fluid through air vent 150 to the atmosphere may be influenced by pressure within the flow path of fluid 130 relative to the atmosphere and the arrangement of the air vent 155, e.g. the number and/or size of orifices 153 and/or openings 156 of the cover 155 etc. The assembly 300 comprises a source of compressed air 310, e.g. an inflator 120 (e.g. pressure in the range of 0 to 500mgHg), a manometer 320 configured to indicate the pressure in the flow path of fluid 130, a flowmeter 330 configured to measure the volume flow of fluid, an air vent 150 and a pneumatic connection 340, e.g. such as a hose, connecting the first end 151 to the second end 152 of the air vent 150. Fluid in the assembly 300 may flow from the source of compressed air 310 through the first end 151 and the second end 152 into the air vent body. The fluid may then flow from the air vent body through cover 155 to the atmosphere.

Fig. 7B shows a diagram showing the flow rate at the orifice inlet in ml/s in dependency of the pressure at the orifice inlet in mmHg, as measured by a prototype of the disclosed air vent.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. "Multiple" elements do include two elements or more. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Air vent (150) for a hemodynamic measurement apparatus (200) comprising an inflatable cuff (110) and an inflator (120), comprising
- an air vent body (160) configured to be disposed in a flow path of fluid (130) between the inflatable cuff (110) and the inflator (120) or at a bladder (112) of the inflatable cuff (110), the air vent body (160) having one or multiple orifices (153) arranged in the air vent body (160), wherein the one or multiple orifices (153) are configured to let fluid pass from the flow path of fluid (130) or from the bladder (112); and
- a cover (155) covering the one or multiple orifices (153) and being configured to permanently let fluid pass from the one or multiple orifices (153) to the atmosphere, wherein the cover (155) is configured and arranged to redirect the flow of fluid from the one or multiple orifices (153) and to protect the one or multiple orifices (153) from material entering the one or multiple orifices (153) from the atmosphere.

2. Air vent (150) according to claim 1,
wherein the cover (155) and the one or multiple orifices (153) are configured and arranged to reduce noise caused by the flow of fluid though the air vent (155) to the atmosphere.

3. Air vent (150) according to any one of the preceding claims,
wherein the cover (155) at least partially encloses the air vent body (160) and comprises a plurality of pores (161) permeable for fluid.

4. Air vent (150) according to any one of the preceding claims,
wherein the cover (155) is arranged at a distance from the air vent body (160) to form one or multiple openings (156) between the air vent body (160) and the cover (155) letting the fluid pass to the atmosphere.

5. Air vent (150) according to any one of the preceding claims,
wherein the air vent body (160) is configured as hollow cylinder or pipe and the cover (155) is ring-shaped and arranged around the air vent body (160).

6. Air vent (150) according to claim 5,
wherein the multiple orifices (153) are distributed over the circumference of the air vent body (160).

7. Air vent (150) according to claim 5 or 6,
wherein at least some of the axes (162) of the multiple orifices (153) intersect in one point of the air vent body (160), in particular in a center point (154) of the air vent body (160), and/or are inclined at different angles with respect to a longitudinal axis (163) of the air vent body (160).

8. Air vent (150) according to any one of claims 5 to 7,
further comprising one or more spacers (157) between the air vent body (160) and the ring-shaped cover (155) to form one or multiple channels (158) between the outer surface of the air vent body (160) and the cover (155).

9. Air vent (150) according to any one of claim 5 to 7,
wherein the ring-shaped cover (155) is arranged in contact with the outer surface of the air vent body (160) and is made of porous material.

10. Air vent (150) according to any one of claims 1 to 4,
wherein the air vent body (160) is configured as a plate or ring segment and the cover (155) is configured as a porous block arranged on top of the air vent body (160).

11. Air vent (150) according to claims 5 to 10,
further comprising a first connector (159A) arranged at a first end (151) of the air vent (150) and a second connector (159B) arranged at a second end (152) of the air vent (150), the first connector (159A) and second connectors (159B) being configured to insert the air vent (150) into the flow path of fluid (130).

12. Air vent (150) according to any one of the preceding claims,
wherein the air vent (150) is configured to be attached to one of an inlet hose (111) of the inflatable cuff (110), a hose (140, 141, 142) configured to connect the inflatable cuff (110) to the inflator (120),
a hose (140, 141, 142) configured to connect the inflatable cuff (110) to the inflator (120) via a patient monitor,
an outlet hose (121) of the inflator (120) or
a fluid connection within the inflator (120).

13. Hemodynamic measurement apparatus (200) comprising:
- an inflatable cuff (110);
- a hose (140, 141, 142) configured to connect the inflatable cuff (110) with an inflator (120); and
- an air vent (150) according to any one of the preceding claims.

14. Hemodynamic measurement apparatus (200) according to claim 13,
wherein the inflatable cuff (110) is a shell cuff, and wherein the air vent (150) is attached to the hose or at the bladder (112) of the inflatable cuff (110).

15. Hemodynamic measurement system (100) comprising:
- a hemodynamic measurement apparatus (200) according to claim 13 or 14; and
- an inflator (120) configured to inflate the inflatable cuff (110).
